# NOUVEAU FASCICULE DE BREVET EUROPEEN

(11) **EP 3 622 946 B2**
(45) Date de publication et mention de la décision concernant l'opposition: **27.09.2023**
(45) Mention de la délivrance du brevet: 06.01.2021
(21) Numéro de dépôt: 19196092.1
(22) Date de dépôt: 09.09.2019
(51) Int. Cl.: A61K 8/44, A61Q 5/02, A61K 8/60, A61Q 5/12, A61K 8/73, A61K 8/92, A61K 8/9794, A61K 8/06

(54) **COMPOSITION COSMETIQUE CAPILLAIRE A BASE D'ALOE VERA ET D'HUILE DE NOIX DE COCO ET SON PROCEDE DE PREPARATION**
HAARKOSMETISCHE ZUSAMMENSETZUNG AUF DER BASIS VON ALOE VERA UND KOKOSNUSSÖL, UND IHR HERSTELLUNGSVERFAHREN
COSMETIC HAIR COMPOSITION MADE OF ALOE VERA AND COCONUT OIL AND METHOD FOR PREPARING SAME

(30) Priorité: 12.09.2018 FR 1871026
(43) Date de publication de la demande: 18.03.2020
(73) Titulaire: Laboratoires Léa, 17183 Perigny (FR)
(72) Inventeur: BOVAËRE, Sarah, 17220 CROIX-CHAPEAU (FR); DABIN, Nathalie, 17220 LA JARRIE (FR); DEMARS MARCADET, Valérie, 17000 LA ROCHELLE (FR)
(74) Mandataire: Axe PI

(56) Documents cités:
- WO-A1-2015/124377
- FR-A1- 2 854 066
- US-A1- 2009 130 220
- US-B1- 9 107 839

## Description

### Domaine Technique

La présente invention concerne le domaine de la cosmétique. Elle concerne plus particulièrement une nouvelle composition cosmétique destinée à être appliquée sur les cheveux et le cuir chevelu comprenant des extraits d'aloé vera (*Aloe barbadensis*) et de fruit de cocotier (*Cocos nucifera*)*,* un procédé de préparation d'une telle composition ainsi que son utilisation pour le lavage ou le soin des cheveux.

Le cheveu est composé de trois couches superposées. A l'extérieur, la cuticule consiste en une fine enveloppe de protection ; au milieu, le cortex, « écorce » intermédiaire, est la partie la plus robuste du cheveu, et représente environ 90% de son poids total ; et à l'intérieur, on trouve la moëlle.

La cuticule forme une gaine épaisse par superposition de nombreuses écailles se recouvrant partiellement. La cuticule est plus particulièrement composée d'écailles, faites de plaques de kératine minces et ultra plates, empilées les unes sur les autres, à raison de plusieurs couches étroitement juxtaposées, à la manière des tuiles d'un toit. Verticalement, ces écailles s'imbriquent tête-bêche dans d'autres écailles, celles de la gaine interne du follicule pileux, tournées vers le haut. Leur imbrication les unes dans les autres permet à la tige pilaire de remonter, et de croître le long du follicule. Horizontalement, les écailles de la cuticule sont reliées entre elles par des céramides, substances huileuses et précieuses, principalement constituées d'acides gras essentiels.

Les cellules de la cuticule sont composées de trois couches : l'endocuticule (la plus profonde), l'exocuticule et l'épicuticule.

L'épicuticule, particulièrement d'intérêt, correspond à la fine couche lipidique à la surface du cheveu. C'est elle qui le rend imperméable, à condition d'être totalement intacte et non-endommagée. Elle représente en moyenne 10% du diamètre du cheveu.

Le cortex est la partie la plus volumineuse et le cœur de la fibre. C'est dans le cortex que sont localisées les protéines fibreuses caractéristiques des cheveux : les a-kératines. La kératine est constituée de deux sortes de fibres, les unes verticales, les autres horizontales. Verticalement, on trouve de multiples écheveaux de protéines, encastrés les uns dans les autres, à savoir des macrofibrilles, comprenant également les pigments de mélanine qui déterminent la couleur du cheveu, lesquelles sont elles-mêmes faites de plusieurs milliers de microfibrilles, lesquelles sont à leur tour assemblées en protofibrilles (fibrilles primitives). Les chaines torsadées suivent l'axe du cheveu et lui confèrent son élasticité. Horizontalement, ces chaines de protéines sont scellées les unes aux autres par des ponts disulfures. Ces ponts confèrent aux chaînes leur rigidité et leur cohérence. Le cortex donne au cheveu sa couleur et sa résistance. Il représente en moyenne 75% du diamètre du cheveu.

La moëlle se situe au centre des cheveux. Elle ressemble à un grand tuyau et est constituée de cellules sans noyau. Elle n'a pas de fonction particulière dans les cheveux humains et ne contribue pas aux propriétés mécaniques. La moelle représente en moyenne 15% du diamètre.

### Technique antérieure

Il existe à ce jour de très nombreux produits cosmétiques tels que des shampooings à base d'extraits végétaux pour le lavage et le soin des cheveux.

L'aloé vera est par exemple largement utilisé en cosmétique pour ses propriétés hydratante, anti-inflammatoire, cicatrisante et son action anti-âge.

Les extraits de noix de coco tels que l'huile de noix de coco sont également couramment utilisés pour nourrir le cheveu.

On connait par exemple le shampooing CLEAR SCALP & HAIR BEAUTY THERAPY INTENSE HYDRATION^{®} de UNILEVER qui comprend entre autres de la poudre de feuilles d'aloé vera (*Aloe barbadensis*), de l'huile de noix de coco (*Cocos nucifera*)*,* ainsi qu'au moins un tensioactif cationique, un tensioactif soufré (TEA-dodecylbenzenesulfonate, TEA-sulfate) et un gélifiant anionique.

De même, on connait le shampooing 365 EVERYDAY VALUE CITRUS GRAPEFRUIT^{®} de WHOLE FOODS MARKET qui comprend notamment du jus de feuilles d'aloé vera (*Aloe barbadensis*)*,* de l'huile de noix de coco (*Cocos nucifera*), ainsi qu'au moins un tensioactif cationique, un tensioactif soufré (isethionate, cocamidopropyl hydroxysultaine) et un gélifiant anionique.

Les tensioactifs soufrés anioniques sont des molécules amphiphiles contenant au moins un atome de soufre. La quasi-totalité des tensioactifs soufrés est regroupée parmi les sulfates et leurs dérivés sulfosuccinate, sulfoacetate, isethionate, taurate.

Les tensioactifs soufrés sont communément utilisés pour leurs très bonnes propriétés moussantes, détergentes et leurs prix compétitifs.

Cependant, ces tensioactifs possèdent une charge négative très importante. Cette charge peut se lier à des sites actifs spécifiques de la séquence protéïque de l'a-kératine (chargée positivement) qui compose le cheveu humain à environ 80%. C'est le cas, par exemple, avec la glycine, un acide aminé chargé positivement, dont les sulfates entrainent la dénaturation. Cette dénaturation va dissoudre du matériel cellulaire et peut aller jusqu'à la dissolution de l'endocuticule pouvant entrainer ensuite le détachement des cuticules lors du prochain shampooing.

En outre, bien que les consommateurs associent souvent la mousse à un produit « lavant », les sulfates sont des tensioactifs agressifs et irritants pour la peau. Ils peuvent altérer le cuir chevelu et provoquer des irritations, démangeaisons ou l'apparition de pellicules. Les irritations peuvent également entrainer un dérèglement des glandes sébacés engendrant une hyperséborrhée.

### Problème technique

Considérant ce qui précède, un problème que se propose de résoudre l'invention est de développer un produit cosmétique destiné à être appliqué sur les cheveux et le cuir chevelu associant un complexe d'ingrédients actifs qui soit le moins agressif possible et qui réponde aux besoins de la fibre capillaire tout en conservant une bonne stabilité et des propriétés moussantes satisfaisantes en termes de volume, de qualité et de rinçage. Plus particulièrement, l'invention a pour but de fournir un produit apportant un actif hydratant, un actif nourrissant et deux catégories de matières propres aux produits moussants (tensioactif et conditionneur) pour conférer de la brillance, de la douceur, du gainage et de la souplesse aux cheveux sans altérer la couche lipidique, appelée épicuticule, de la fibre capillaire.

### Solution technique

La solution à ce problème posé a pour premier objet une composition cosmétique destinée à être appliquée sur les cheveux et le cuir chevelu comprenant des extraits d'aloé vera (*Aloe barbadensis*) et de fruit de cocotier (*Cocos nucifera*)*,* caractérisée en ce qu'elle se présente sous forme d'une émulsion moussante comprenant, dans un milieu cosmétiquement acceptable :
- un extrait d'aloé vera choisi parmi une eau, un jus et/ou de la poudre d'aloé vera ;
- entre 1% et 20% en poids du poids total de la composition (p/p) d'une huile de noix de coco ;
- au moins un tensioactif non soufré et non éthoxylé ; et ne comprend pas de tensioactif soufré et/ou éthoxylé ; et
- au moins un agent conditionneur cationique issu de gomme guar (*Cyamopsis tetragonoloba*).

Elle a pour deuxième objet un procédé de préparation d'une composition selon l'invention, caractérisé en ce qu'il comprend les étapes suivantes selon lesquelles :
a) on mélange le ou les extraits d'aloé vera choisi(s) parmi une eau, un jus et/ou de la poudre d'aloé vera dans de l'eau,
b) on disperse au moins un agent conditionneur cationique issu de gomme guar *(Cyamopsis tetragonoloba*) dans le mélange obtenu en a),
c) après neutralisation, on laisse un gel aqueux se former par agitation supérieure à 1000 tours/minute sous émulseur,
d) éventuellement, on disperse un gélifiant anionique par agitation supérieure à 1000 tours/minute sous émulseur et chauffage à une température supérieure à 50°C,
e) entre 1% et 20% d'huile de noix de coco en poids du poids total de la composition (p/p) est chauffé à une température supérieure à 50°C puis est ajouté au gel aqueux obtenu en c) ou d) sous émulseur, et
f) un tensioactif non soufré et non éthoxylé est ajouté à une température inférieure à 30°C sous agitation inférieure à 1000 tours/minutes pour obtenir une émulsion moussante.

Elle a pour troisième objet l'utilisation cosmétique d'une composition selon l'invention pour le lavage ou le soin des cheveux.

Enfin, l'invention a pour dernier objet un procédé de traitement cosmétique des cheveux caractérisé en ce qu'il consiste à appliquer sur les cheveux une quantité efficace d'une composition selon l'invention puis à effectuer éventuellement un rinçage à l'eau.

### Avantages apportés

La composition cosmétique selon l'invention comprend une association synergique :
- d'aloé vera permettant d'hydrater intensément la fibre capillaire et le cuir chevelu et d'apaiser les cuirs chevelus irrités et les démangeaisons. Ses nutriments vont également apporter de la force, de la brillance et de l'éclat aux cheveux, les rendant ainsi moins cassants et plus lisses ;
- d'une huile de noix de coco, pour sa richesse notamment en acide laurique lui permettant de pénétrer la fibre capillaire pour une nutrition en profondeur ;
- de tensioactif(s) non soufré(s) et non éthoxylé(s), pour la mousse qu'ils apportent sans dénaturer le cheveu, ni irriter le cuir chevelu ; et
- d'agent(s) conditionneur(s) cationique(s) issu(s) de gomme guar, pour leur action gainante, n'étouffant pas le cheveu et laissant ainsi le complexe d'actifs pénétrer.

Aussi, la réalisation d'une émulsion moussante selon l'invention permet d'avoir des quantité et qualité de mousse satisfaisantes malgré l'ajout d'huile de coco qui va venir « casser » la mousse et l'absence de tensioactifs soufrés réputés pour leurs qualités moussantes. L'ajout d'huile(s) végétale(s) dans une émulsion moussante va, en effet, limiter considérablement l'effet moussant de celle-ci.

Or, il est connu que les tensioactifs étant généralement des matières aqueuses, le rajout d'une grande quantité d'eau après une émulsion vient troubler l'équilibre des phases et va déstabiliser totalement cette dernière, ne permettant pas sa commercialisation.

Pour lever cette contrainte, le choix des tensioactifs utilisés selon l'invention ainsi que le mode opératoire réalisé est essentiel pour obtenir le bon compromis entre mousse et stabilité de la composition tout en conservant leurs propriétés détergentes.

En outre, la mise en œuvre d'une telle composition selon l'invention permet de combiner une gomme guar cationique avec un gélifiant anionique, en théorie incompatibles.

Enfin, d'un point de vue règlementaire et écologique, la composition selon l'invention répond au label COSMOS Organic certifié par le groupe ECOCERT n'autorisant que des matières respectant l'environnement. Par ailleurs, elle ne contient aucun dérivé d'huile de palme pour des raisons éthiques et environnementales.

Dans cette description, à moins qu'il n'en soit spécifié autrement, il est entendu que, lorsqu'un intervalle est donné, il inclut les bornes supérieure et inférieure dudit intervalle.

### Brève description des dessins

L'invention et les avantages qui en découlent seront mieux compris à la lecture de la description et des modes de réalisation non limitatifs qui suivent, illustrés au regard des dessins annexés dans lesquels :
La Figure 1 représente une image (grossissement *1000) de microscopie électronique à balayage (MEB) de cuticules d'un cheveu mettant en évidence les effets d'une composition selon l'invention utilisée comme «témoin» (Référence 2357A.21) mise en œuvre dans le cadre de l'exemple 2.
La Figure 2 représente une image MEB (grossissement *1000) de cuticules d'un cheveu mettant en évidence les effets de la composition « témoin » selon l'invention ne comprenant pas de gomme guar quaternisée (Référence 2357A.22) mise en œuvre dans le cadre de l'exemple 2.
La Figure 3 représente une image MEB (grossissement *1000) de cuticules d'un cheveu mettant en évidence les effets de la composition «témoin» selon l'invention dans laquelle la gomme guar quaternisée a été remplacée par un ammonium quaternaire de synthèse, à savoir le cetrimonium chloride (Référence 2357A.23), mise en œuvre dans le cadre de l'exemple 2.
La Figure 4 représente une image MEB (grossissement *1000) de cuticules d'un cheveu mettant en évidence les effets de la composition « témoin » selon l'invention ne comprenant pas d'huile de coco (Référence 2357A.24) mise en œuvre dans le cadre de l'exemple 2.
La Figure 5 représente une image MEB (grossissement *1000) de cuticules d'un cheveu mettant en évidence les effets de la composition «témoin» selon l'invention comprenant 0,5% d'huile de coco (Référence 2357A.25) mise en œuvre dans le cadre de l'exemple 2.
La Figure 6 représente une image MEB (grossissement *1000) de cuticules d'un cheveu mettant en évidence les effets de la composition «témoin» selon l'invention comprenant 1% d'huile de coco (Référence 2357A.26) mise en œuvre dans le cadre de l'exemple 2.
La Figure 7 représente une image MEB (grossissement *1000) d'un échantillon de cuticules d'un cheveu mettant en évidence les effets de la composition « témoin » selon l'invention ne comprenant pas d'aloé vera (Référence 2357A.27) mise en œuvre dans le cadre de l'exemple 2.
La Figure 8 représente une image MEB (grossissement *1000) de cuticules d'un cheveu mettant en évidence les effets de la composition « témoin » selon l'invention comprenant un tensioactif soufré (Référence 2357A.28) mise en œuvre dans le cadre de l'exemple 2.

### Description des modes de réalisation

L'invention concerne une composition cosmétique destinée à être appliquée sur les cheveux et le cuir chevelu comprenant des extraits d'aloé vera (*Aloe barbadensis*) et de fruit de cocotier (*Cocos nucifera*). Elle se présente sous forme d'une émulsion moussante comprenant, dans un milieu cosmétiquement acceptable :
- un extrait d'aloé vera choisi parmi une eau, un jus et/ou de la poudre d'aloé vera ;
- entre 1% et 20% en poids du poids total de la composition (p/p) d'une huile de noix de coco ;
- au moins un tensioactif non soufré et non éthoxylé ; et ne comprend pas de tensioactif soufré et/ou éthoxylé ; et
- au moins un agent conditionneur cationique issu de gomme guar (*Cyamopsis tetragonoloba*).

La composition selon l'invention comprend un extrait d'aloé vera choisi parmi une eau, un jus et/ou de la poudre d'aloé vera.

L'aloé vera est une plante xérophyte et succulente, c'est-à-dire qu'elle pousse en milieu aride et stocke l'eau dans ses feuilles. L'eau est donc le principal constituant de ces feuilles (98 à 99%). Les 1 à 2% restants sont de la matière sèche, très riche en composants nutritionnels.

Ces composants sont notamment :
- des vitamines : A, B1, B2, B3, B6, B9 et B12 ;
- des minéraux : calcium, chlore, chrome, cuivre, fer, magnésium, manganèse, phosphore, potassium, sodium, zinc ;
- des acides aminés essentiels : Isoleucine, leucine, lysine, méthionine, phénylalanine, thréonine, valine ;
- des acides aminés secondaires : acide aspartique, acide glutamique, alanine, arginine, cystine, glycine, histidine, hydroxiproline, proline, sérine, tyrosine ;
- des enzymes anti-inflammatoires ;
- des mono- et polysacharrides : cellulose, glucose, galactose, mannose, aldonentose, acide uronique, lipase, aliinase, L-rhamnose ; et
- de la lignine, des saponines et anthraquinones.

La synergie de ces vitamines, minéraux, enzymes et autres acides aminés confère à l'aloé vera des propriétés hydratantes, anti-inflammatoire, cicatrisante et une action anti-âge.

Plus particulièrement, l'extrait d'aloé vera va hydrater intensément la fibre capillaire et apporter de la force, de la brillance et de l'éclat aux cheveux, les rendant ainsi moins cassants et plus lisses.

Il va également hydrater le cuir chevelu, ce qui peut apaiser les cuirs chevelus irrités et les démangeaisons.

De manière avantageuse, les enzymes présentent dans l'aloé vera peuvent également prévenir la chute des cheveux en protégeant le cuir chevelu des agressions, et son pH légèrement acide va faciliter la pénétration des nutriments qui vont pouvoir revitaliser le bulbe, le renforcer et ainsi stimuler la pousse des cheveux.

L'extrait d'aloé vera de la composition selon l'invention est préférentiellement de la poudre de feuilles d'aloé vera et/ou du jus de feuilles d'aloé vera, plus préférentiellement un mélange de poudre de feuilles d'aloé vera et de jus de feuilles d'aloé vera.

La composition selon l'invention comprend préférentiellement de la poudre de feuilles d'aloé vera à une concentration comprise entre 0,01% et 2% en poids du poids total de la composition (p/p) et/ou du jus de feuilles d'aloé vera à une concentration comprise entre 0,5% et 15% en poids du poids total de la composition (p/p).

La composition selon l'invention comprend plus préférentiellement de la poudre de feuilles d'aloé vera à une concentration comprise entre 0,01% et 2% en poids du poids total de la composition (p/p) et du jus de feuilles d'aloé vera à une concentration comprise entre 0,5% et 15% en poids du poids total de la composition (p/p).

Préférentiellement, la composition selon l'invention comprend de la poudre de feuilles d'aloé vera à une concentration comprise entre 0,02% et 0,5% en poids du poids total de la composition (p/p), par exemple 0,03%, 0,04%, 0,04%, 0,05%, 0,06%, 0,07%, 0,08%, 0,09%, 0,1%, 0,2%, 0,3% ou encore 0,4%.

Préférentiellement, la composition selon l'invention comprend du jus de feuilles d'aloé vera à une concentration comprise entre 1% et 10% en poids du poids total de la composition (p/p), par exemple 2%, 3%, 4%, 5%, 6%, 7%, 8% ou encore 9%.

La composition selon l'invention comprend également entre 1% et 20% en poids du poids total de la composition (p/p) d'une huile de noix de coco.

L'huile de coco est composée de lipides et d'insaponifiables (terpénoïdes et vitamines). Les lipides, très majoritaires dans la composition de l'huile de noix de coco, sont présents sous la forme d'acides gras et de phospholipides. Les acides gras composant l'huile de noix de coco sont :
- l'acide laurique (40 à 55%) ;
- l'acide myristique (15 à 23%) ;
- l'acide oléique (5 à 15%) ;
- l'acide palmitique (5 à 12%) ;
- l'acide caprylique (5 à 10%) ;
- l'acide caprique (5 à 9%) ;
- l'acide stéarique (1 à 4%) ;
- l'acide linoléique (1 à 2%) ; et
- les acides palmitoléique, caproïque, eicosénoïque, linolénique et arachidique (traces).

L'huile de noix de coco comprenant une forte concentration en acide laurique a la capacité de pénétrer la fibre capillaire. En effet, l'acide laurique a une grande affinité avec les protéines du cheveux et grâce à son faible poids moléculaire et sa chaine linéaire, est capable de pénétrer la fibre capillaire.

L'huile de noix de coco confère à la fibre capillaire des propriétés gainantes, protectrices, restructurantes et permet également de diminuer la pénétration de l'eau dans le cortex, évitant ainsi le gonflement de la fibre.

L'huile de noix de coco permet ainsi d'obtenir des cheveux doux, soyeux, souples, disciplinés, brillants, embellis, gainés, éclatants, hydratés, légers, protégés, réparés, sublimés, revitalisés, fortifiés, restructurés, nourris, renforcés et régénérés.

Les cheveux ont également du volume et de la vitalité, et les pointes sont moins sèches.

De manière particulièrement avantageuse, l'huile de noix de coco facilite le démêlage et améliore la sensation d'hydratation des cheveux; le cuir chevelu est également hydraté.

La composition selon l'invention comprend de l'huile de noix coco à une concentration comprise entre 1% et 20% en poids du poids total de la composition (p/p).

La composition selon l'invention comprend plus préférentiellement de l'huile de noix coco à une concentration comprise entre 1% et 10% en poids du poids total de la composition (p/p), par exemple de 1,5%, 2%, 2,5%, 3%, 3,5%, 4%, 4,5%, 5%, 5,5%, 6%, 6,5%, 7%, 7,5%, 8%, 8,5%, 9% ou encore 9,5%.

La quantité d'huile de noix de coco comprise dans la composition selon l'invention oblige à réaliser une émulsion. La contrainte technique est alors de réaliser une émulsion moussante, c'est-à-dire de réussir à combiner les tensioactifs apportant la mousse à l'émulsion.

En effet, les tensioactifs sont des matières aqueuses qui sont, en plus, dispersées dans l'eau. Le rajout d'une grande quantité d'eau après une émulsion vient troubler l'équilibre des phases et va déstabiliser totalement l'émulsion, ne permettant pas la commercialisation du produit fini.

Le choix des tensioactifs compris dans la composition selon l'invention est donc essentiel et comprend au moins un tensioactif non soufré et non éthoxylé.

La composition selon l'invention ne comprend pas de tensioactif soufré et/ou éthoxylé.

Outre les inconvénients préalablement décrits des tensioactifs soufrés, la synthèse de tensioactif éthoxylé est toxique et polluante pour l'environnement.

Le tensioactif non soufré et non éthoxylé compris dans la composition selon l'invention est préférentiellement choisi parmi la cocamidopropyl betaine, le decyl glucoside, le sodium cocoyl glutamate, le lauryl glucoside, le coco glucoside, le sodium lauroyl glutamate, le sodium olivoyl glutamate, et les tensioactifs dérivés de glucamide tels que le cocoyl méthyle glucamide ou le sunfloweroyl méthyle glucamide, pris seul ou en mélange.

Préférentiellement, la composition selon l'invention comprend un tensioactif non soufré et non éthoxylé pris seul ou en mélange à une concentration comprise entre 0,5% et 40% en poids du poids total de la composition (p/p), plus préférentiellement entre 1% et 20%, par exemple 2%, 5%, 10% ou encore 15%.

Préférentiellement, le tensioactif non soufré et non éthoxylé compris dans la composition selon l'invention est choisi parmi la cocamidopropyl betaine, le decyl glucoside, ou le sodium cocoyl glutamate, pris seul ou en mélange.

La composition selon l'invention comprend avantageusement entre 0,5 et 20% (p/p) de cocamidopropyl betaine, entre 0,5% et 20% (p/p) de decyl glucoside et/ou entre 0,25% et 10% (p/p) de sodium cocoyl glutamate.

Plus préférentiellement, le tensioactif non soufré et non éthoxylé compris dans la composition selon l'invention est un mélange de cocamidopropyl betaine, decyl glucoside et sodium cocoyl glutamate.

Encore plus préférentiellement, la composition selon l'invention comprend respectivement, en combinaison, entre 0,5 et 20% (p/p) de cocamidopropyl betaine, par exemple entre 0,5 et 10%, entre 0,5% et 20% (p/p) de decyl glucoside, par exemple entre 0,5% et 10%, et entre 0,25% et 10% (p/p) de sodium cocoyl glutamate, par exemple entre 0,25% et 7%.

Le choix de tel(s) tensioactif(s) non soufré(s) et non éthoxylé(s) permet de stabiliser l'émulsion malgré l'ajout d'une grande quantité de matières aqueuses, et également de trouver un compromis entre stabilité de l'émulsion et quantité de mousse dans le produit fini.

Les tensioactifs doivent être ajoutés sous agitation douce, avantageusement sous agitation inférieure à 1000 tours/minutes, à froid, à une température inférieure à 30°C, avantageusement de l'ordre de 20°C, une fois l'émulsion réalisée. Ils vont, dans le cas contraire, faire mousser l'émulsion donc gêner sa réalisation et ils seront eux même pris dans cette émulsion, ce qui limite considérablement leurs propriétés moussantes.

La composition selon l'invention comprend également au moins un agent conditionneur cationique issu de gomme guar (*Cyamopsis tetragonoloba*).

La guar (*Cyamopsis tetragonoloba*) est une plante cultivée, entre autres, en Afrique de l'ouest, au Pakistan et en Inde. Cette plante produit des graines qui sont décortiquées, broyées et tamisées pour obtenir une gomme. Il s'agit de la gomme de guar, composée essentiellement de galactomannane, c'est-à-dire d'une chaine de monomères de mannose à laquelle une unité de galactose est ramifiée par un pont en 1-6.

L'agent conditionneur cationique issu de gomme guar compris dans la composition selon l'invention est préférentiellement un dérivé de gomme guar quaternisée, plus préférentiellement un chlorure de guar hydroxypropyltriméthylammonium.

La gomme guar naturelle est ainsi préférentiellement couplée à du 3-chloro-2-hydroxypropyl trimethylammonium chloride afin d'obtenir une gomme guar quaternisée chargée positivement.

Le greffage de cette partie cationique fait de la gomme guar un excellent conditionneur pour les cheveux. En effet, la charge positive de la gomme guar quaternisée va se fixer sur les charges négatives du cheveu.

Plus le cheveu est abimé et plus la charge négative est importante entrainant alors des cheveux électriques et emmêlés.

L'agent conditionneur cationique issu de gomme guar compris dans la composition selon l'invention va donc se fixer spécifiquement sur les sites endommagés grâce à sa charge positive et va ensuite permettre de démêler, de réduire l'électricité statique et d'apporter de la douceur grâce aux longues chaines de galactomannanes.

Le fait qu'il sélectionne l'endroit où il se fixe va limiter le risque de cheveux lourds et ternes comme pourrait le faire par exemple des silicones. De plus, il est non substantif et s'élimine donc facilement lors du shampooing suivant, contrairement, une nouvelle fois, aux silicones.

L'agent conditionneur cationique issu de gomme guar compris dans la composition selon l'invention va permettre de démêler les cheveux sans les abimer, ni les alourdir, optimisant ainsi l'action du complexe huile de coco-aloé vera.

La composition selon l'invention comprend préférentiellement l'agent conditionneur cationique issu de gomme guar à une concentration comprise entre 0,05% et 1% en poids du poids total de la composition (p/p), plus préférentiellement entre 0,1% et 0,8%, par exemple 0,2%, 0,3%, 0,4%, 0,5%, 0,6% ou encore 0,7%.

Selon un mode de réalisation particulièrement avantageux de la composition selon l'invention, elle comprend en outre au moins un gélifiant anionique choisi parmi la gomme xanthane, la gomme caroube, la gomme gellane, la cellulose, la pectine, la bentonite, les carraghénanes, préférentiellement la gomme xanthane.

Le gélifiant anionique permet d'épaissir et de stabiliser l'émulsion selon l'invention.

L'utilisation d'un tel gélifiant anionique, chargé négativement, est en théorie totalement incompatible avec la gomme guar cationique, chargée positivement.

Pour ce faire, les ratios utilisés entre ces deux types d'ingrédients sont essentiels. Dans la mesure où la gomme guar quaternisée est avantageusement utilisée à une concentration de 0,05-1%, en poids du poids total de la composition (p/p), il est nécessaire d'utiliser le gélifiant anionique, préférentiellement la gomme xanthane, à une concentration de 0,1-2% en poids du poids total de la composition (p/p).

Le mode d'incorporation (temps d'agitation, température, ...) de ces deux types d'ingrédients est également important pour améliorer leur compatibilité tel que décrit ci-après dans le procédé de préparation selon l'invention.

Préférentiellement, la composition selon l'invention comprend l'agent gélifiant anionique à une concentration comprise entre 0,1% et 2% en poids du poids total de la composition (p/p).

Un milieu cosmétiquement acceptable désigne un milieu adapté pour une utilisation en contact avec des cellules humaines et animales, en particulier les cellules de la peau, le cuir chevelu et les cheveux, sans toxicité, irritation, réponse allergique indue et similaires, et proportionné à un rapport avantage/risque raisonnable.

Un tel milieu cosmétiquement acceptable peut comprendre d'autres ingrédients adaptés, connus et utilisés dans le domaine cosmétique. Dans tous les cas, l'homme du métier veillera à choisir le milieu cosmétiquement acceptable (choix des ingrédients additionnels ainsi que leurs proportions) de manière à ce qu'il ne nuise pas aux propriétés avantageuses recherchées de la composition selon l'invention.

A titre d'exemple non limitatif d'ingrédient additionnel, la composition selon peut comprendre en outre un agent hydratant ou apaisant choisi parmi l'acide hyaluronique, le sodium PCA, la glycérine et autres dérivés du glycérol ou un polysaccharide tel qu'un polysaccharide riche en galactomannanes, par exemple la gomme tara (*caesalpina spinosa*).

La composition selon l'invention peut également comprendre en outre une autre huile végétale choisie parmi l'huile de ricin, argan, jojoba, amande douce, karité, avocat, carotte, noyaux d'abricot, germe de blé, nigelle, moutarde, bourrache, noisette, macadamia, abyssinie, sésame, onagre, figue de barbarie, pépins de raisin, carthame, cameline, karanja, camélia, chia, lin, mangue, baobab, brocolis, andiroba, babassu, dattier du désert, monoï, moringa, prise seule ou en mélange.

La composition selon l'invention peut également comprendre en outre un autre agent conditionneur capillaire choisi parmi l'hydroxypropyltrimonium honey, le starch hydroxypropyltrimonium chloride, le brassicamodipropyl dimethylamine, le brassicyl isoleucinate esylate et le brassicyl valinate esylate, pris seul ou en mélange.

Les compositions selon l'invention se présentent sous une forme adaptée à l'application par voie topique, sans risque d'inconfort lors de leur application.

Par application topique, on désigne plus particulièrement le fait d'appliquer ou d'étaler la composition selon l'invention, sur les cheveux et le cuir chevelu.

Les compositions topiques pour la mise en œuvre de l'invention pourront notamment se présenter sous forme de produit à rincer tel qu'un shampooing, après-shampooing, masque, ou encore de produit sans rinçage tel qu'une crème capillaire, sérum hydratant.

Un autre objet de l'invention concerne un procédé de préparation d'une composition selon l'invention comprenant les étapes suivantes selon lesquelles :
a) on mélange le ou les extraits d'aloé vera choisi(s) parmi une eau, un jus et/ou de la poudre d'aloé vera dans de l'eau,
b) on disperse au moins un agent conditionneur cationique issu de gomme guar dans le mélange obtenu en a),
c) après neutralisation, on laisse un gel aqueux se former par agitation supérieure à 1000 tours/minute, par exemple de l'ordre de 1500 tours/minute, sous émulseur,
d) éventuellement, on disperse un gélifiant anionique par agitation supérieure à 1000 tours/minute sous émulseur et chauffage à une température supérieure à 50°C, par exemple par agitation de l'ordre de 2500 tours/minute et chauffage à une température de l'ordre de 70°C,
e) entre 1% et 20% d'huile de noix de coco en poids du poids total de la composition (p/p) est chauffé à une température supérieure à 50°C, avantageusement de l'ordre de 70°C, puis est ajouté au gel aqueux obtenu en c) ou d) sous émulseur, par exemple par agitation de l'ordre de 2500 tours/minute, et
f) un tensioactif non soufré et non éthoxylé est ajouté à froid, à une température inférieure à 30°C, avantageusement de l'ordre de 20°C, sous agitation inférieure à 1000 tours/minutes, par exemple par agitation de l'ordre de 900 tours/minute, pour obtenir une émulsion moussante.

Préférentiellement, on réalise l'étape d) selon laquelle on disperse un gélifiant anionique par agitation supérieure à 1000 tours/minute sous émulseur et chauffage à une température supérieure à 50°C.

Selon un mode de réalisation avantageux de l'invention, une gomme tara (*caesalpina spinosa*) est ajoutée dans une dernière étape.

Selon un mode de réalisation alternatif de la composition selon l'invention, on réalise notamment les étapes suivantes selon lesquelles le gélifiant anionique est dispersé au départ, avec le ou les extraits d'aloé vera choisi(s) parmi une eau, un jus et/ou de la poudre d'aloé vera eux-mêmes dispersés dans de l'eau , le gel de gomme guar quaternisée est également formé seul dans une quantité d'eau à définir selon le pourcentage de guar et enfin on ajoute ce prémélange en fin d'émulsion, en veillant à ne pas déstabiliser celle-ci. Cela permet d'espacer les charges incompatibles et d'améliorer leurs compatibilités.

Un autre objet de l'invention concerne l'utilisation cosmétique d'une composition selon l'invention pour le lavage ou le soin des cheveux.

Un dernier objet de l'invention concerne un procédé de traitement cosmétique des cheveux consistant à appliquer sur les cheveux une quantité efficace d'une composition selon l'invention puis à effectuer éventuellement un rinçage à l'eau.

Avantageusement, un rinçage est réalisé.

Par quantité efficace, on désigne la quantité minimum de composition selon l'invention qui est nécessaire pour obtenir son activité, en particulier cosmétique et plus particulièrement pour le lavage ou le soin des cheveux.

La composition selon l'invention va hydrater intensément la fibre capillaire et apporter de la force, de la brillance et de l'éclat aux cheveux, les rendant ainsi moins cassants et plus lisses.

La composition selon l'invention peut conférer à la fibre capillaire des propriétés gainantes, protectrices, restructurantes et permet ainsi d'obtenir des cheveux doux, soyeux, souples, disciplinés, brillants, embellis, gainés, éclatants, hydratés, légers, protégés, réparés, sublimés, revitalisés, fortifiés, restructurés, nourris, renforcés et régénérés. Les cheveux retrouvent du volume et de la vitalité, et les pointes sont moins sèches et leur démêlage est facilité.

Elle peut permettre également de diminuer la pénétration de l'eau dans le cortex, évitant ainsi le gonflement de la fibre et d'hydrater le cuir chevelu, ce qui peut apaiser les cuirs chevelus irrités et les démangeaisons.

De manière avantageuse, elle peut également permettre de prévenir la chute des cheveux en protégeant le cuir chevelu des agressions, et peut revitaliser le bulbe, le renforcer et ainsi stimuler la pousse des cheveux.

### Exemples

La présente invention va maintenant être illustrée au moyen des exemples suivants :

### Exemple 1 : Préparation d'un exemple de composition selon l'invention Tableau 1 :

| Phase | INCI | Gamme de concentration (% en poids du poids total de la composition) |
|---|---|---|
| A | Ajouter l'eau et les premiers actifs | |
| | AQUA | 50-100% |
| | ALOE BARBADENSIS LEAF JUICE POWDER | 0,01 - 2% |
| | ALOE BARBADENSIS LEAF JUICE | 0,5-15% |
| | SODIUM LEVULINATE | |
| | SODIUM BENZOATE | |
| | CITRIC ACID | |
| | AQUA | |
| | GLYCERIN | |
| B | Ajouter un conservateur | |
| | BENZYL ALCOHOL | 0-1% |
| C | Disperser la gomme guar quaternisée | |
| | GUAR HYDROXYPROPYLTRIMONIUM CHLORIDE | 0,05-1% |
| | AQUA | |
| C' | Neutraliser avec l'acide lactique, et laisser le gel se former sous émulseur pendant 20 min à 1500 tr/min. | |
| | LACTIC ACID | 0,01-2% |
| | AQUA | |
| D | Disperser les gommes, et agiter sous émulseur pendant 10 min à 2500 tr/min. Chauffer à 70°C => vérifier qu'il n'y ait pas de gommes sur l'émulseur. | |
| | XANTHAN GUM | 0,1-2% |
| E | Chauffer la phase E à 70°C puis émulsionner pendant 10 min. | |
| | COCOS NUCIFERA OIL | 1-20% |
| | RICINUS COMMUNIS OIL | 0,1-10% |
| | GLYCERYL STEARATE | 0,1-10% |
| E' | Emulsionner 10 min à 2500tr/min | |
| | AQUA | 0,2-5% |
| | SODIUM COCOYL GLUTAMATE | |
| F | A T<30°C, ajouter sous agitation douce (900 tr/min pendant 10 min). | |
| | AQUA | 0,5-20% |
| | COCAMIDOPROPYL BETAINE | |
| | SODIUM CHLORIDE | |
| G | Solubiliser les parfums dans le decyl glucoside puis ajouter sous agitation douce (900 tr/min pendant 10 min). | |
| | DECYL GLUCOSIDE | 0,5-20% |
| | AQUA | |
| | PARFUM | 0,1-5% |
| | LINALOOL | |
| G' | Ajouter sous agitation douce (900 tr/min pendant 10 min). | |
| | AQUA | 0,25-10% |
| | SODIUM COCOYL GLUTAMATE | |
| H | Ajouter sous agitation douce (900 tr/min pendant 10 min). | |
| | AQUA | / |
| | GLYCERIN | |
| | CAESALPINA SPINOSA GUM | |
| | SODIUM BENZOATE | |
| | POTASSIUM SORBATE | |
| | AQUA | |
| | SODIUM HYALURONATE | |
| I | Ajuster le pH entre 4,0 et 6,0 | |
| | LACTIC ACID AQUA | 0,1-2% |

Selon le procédé avantageux de l'Exemple 1, une première partie du sodium cocoyl glutamate est ajoutée à l'étape E' pour stabiliser et affiner l'émulsion ; le reste du sodium cocoyl glutamate est ensuite ajouté à l'étape G' pour ses propriétés moussantes.

Une gomme tara peut également être avantageusement ajoutée dans une étape additionnelle H.

Différents types de tests ont été réalisés ci-après, dans les mêmes conditions et le même jour pour chaque composition testée. A cet effet, plusieurs émulsions moussantes ont été réalisées pour déterminer l'effet synergique des différents ingrédients de la composition selon l'invention.

**Tableau 2**

| | |
|---|---|
| Essai 2357A.21 | Composition selon l'invention « témoin » comprenant : 5% de jus d'aloé vera, 0,04% de poudre d'aloé vera, 0,185% de gomme guar quaternisée, 1,5% d'huile de noix de coco, et un mélange de 4,736% cocamidopropyl betaine, 5% decyl glucoside, 0,285% sodium cocoyl glutamate |
| Essai 2357A.22 | Composition « témoin » sans gomme guar quaternisée |
| Essai 2357A.23 | Composition « témoin » sans gomme guar quaternisée, remplacée par un ammonium quaternaire de synthèse (cetrimonium chloride) concentré à 0,185% |
| Essai 2357A.24 | Composition « témoin » sans huile de noix de coco |
| Essai 2357A.25 | Composition « témoin » avec 0,5% d'huile de noix de coco |
| Essai 2357A.26 | Composition « témoin » avec 1% d'huile de noix de coco |
| Essai 2357A.27 | Composition « témoin » sans aloé vera (ni jus ni poudre) |
| Essai 2357A.28 pour observation MEB | Composition « témoin » avec le mélange de tensioactifs non soufrés non éthoxylés remplacé par un tensioactif soufré (sodium lauryl sulfate à 10,021 %) |
| Essai 2357A.29 pour tests mousse/ coiffeur | Composition « témoin » avec le mélange de tensioactifs non soufrés non éthoxylés remplacé par un tensioactif soufré (sodium lauryl sulfate à 5,285%) et 4,736% de cocamidopropyl betaine |

Pour chaque composition, la composition selon l'invention «témoin» préparée selon l'exemple 1 a été utilisée comme formulation de base à partir des mêmes lots de matières premières, à laquelle seules les modifications indiquées dans le Tableau 2 ci-dessus ont été apportées.

### Exemple 2 : Microscopie Electronique à Balayage (MEB)

Une observation au microscope à balayage a été réalisée. Cette technique permet d'observer en détails les cuticules d'un cheveu et donc d'évaluer l'effet des différentes compositions capillaires testées.

Les tests ont été réalisés au sein du secteur microscopie électronique de l'Université de La Rochelle. Les observations ont été faites sur le microscope FEI Quanta 200 ESEM/FEG Environnemental, Canon FEG, Résolution 3 nm.

Pour réaliser ces tests, des mèches naturelles européennes, de teinte 9 et de 20 cm de longueur, fournies par la société Kerling Int. Haarfabrik GmbH ont été utilisées.

Ces mèches ont été dénaturées avec une solution dénaturante contenant 25,694% d'eau osmosée, 0,306% de lessive de soude et 74% d'IFRAPON LAM BENZ^{®} fourni par la société IFRACHIMIE S.A.S concentré à 27% en sodium lauryl sulfate. Le pH final de cette solution est de 7,99.

Le protocole de dénaturation des mèches est le suivant :
1. Préparer 500 mL de solution dénaturante dans un bécher.
2. Immerger la mèche dans la solution dénaturante, faire 5 tours de bécher puis laisser la mèche en immersion dans cette solution pendant 3 minutes.
3. Rincer la mèche dans 5 bains remplis de 200 mL d'eau osmosée :
   a. Tourner la mèche 3 fois dans le bécher du 1er bain de rinçage.
   b. Sortir la mèche du bain et la passer entre 2 doigts pour l'essorer.
   c. Recommencer de la même manière dans les 3 bains suivants.
   d. Immerger la mèche dans le 5ème bain de rinçage, la tourner 3 fois puis la laisser immergée pendant 30 secondes avant de l'enlever du bain et de la passer entre 2 doigts pour l'essorer.
4. Ne pas sécher la mèche, et appliquer directement le produit à tester sur mèche mouillée.
   8 mèches de cheveux ont été dénaturées avec ce protocole. Une fois cette dénaturation réalisée, les différentes compositions détaillées dans le Tableau 2 ont été appliquées, une composition étant appliquée par mèche dénaturée.

Le protocole d'application utilisé est le suivant :
1. Déposer 0,5 g de composition à tester dans un verre de montre.
2. Passer une mèche dénaturée dans la composition pour l'enduire.
3. Déposer la mèche dans la main ouverte et faire 10 mouvements circulaires avec l'autre main pour faire mousser.
4. Rincer la mèche immédiatement dans 5 bains remplis de 200 mL d'eau osmosée :
   a. Tourner la mèche 3 fois dans le bécher du 1er bain de rinçage.
   b. Sortir la mèche du bain et la passer entre 2 doigts pour l'essorer.
   c. Recommencer de la même manière dans les 3 bains suivants.
   d. Immerger la mèche dans le 5ème bain de rinçage, la tourner 3 fois puis la laisser immergée pendant 30 secondes avant de l'enlever du bain et de la passer entre 2 doigts pour l'essorer.
5. Démêler la mèche en passant 1 fois le peigne sur cheveux mouillés.
6. Déposer la mèche sur papier absorbant et laisser sécher naturellement.

Ces préparations ont été réalisées le même jour, au laboratoire, à une température ambiante de 23,5°C, la température des bains d'eau osmosée étant de 25°C et le pH de cette eau de 6,21.

Les observations au MEB ont été réalisées le lendemain. Pour ce faire, une partie centrale de la mèche a été découpée et déposée cheveu par cheveu sur des platines destinées à l'observation.

Les observations ont été faites en conditions environnementales, c'est-à-dire sous vapeur d'eau, ce qui rend l'atmosphère autour de l'échantillon conductrice permettant ainsi d'analyser des échantillons biologiques sans les dégrader. Les conditions de pression, tension et températures sont constantes à l'intérieur de la chambre, à savoir P=1,3 mbaret U=10 kV.

Les résultats obtenus (photographies au grossissement *1000) au global sont détaillés ci-après, 3 mèches de cheveux ayant été observées pour chaque essai.

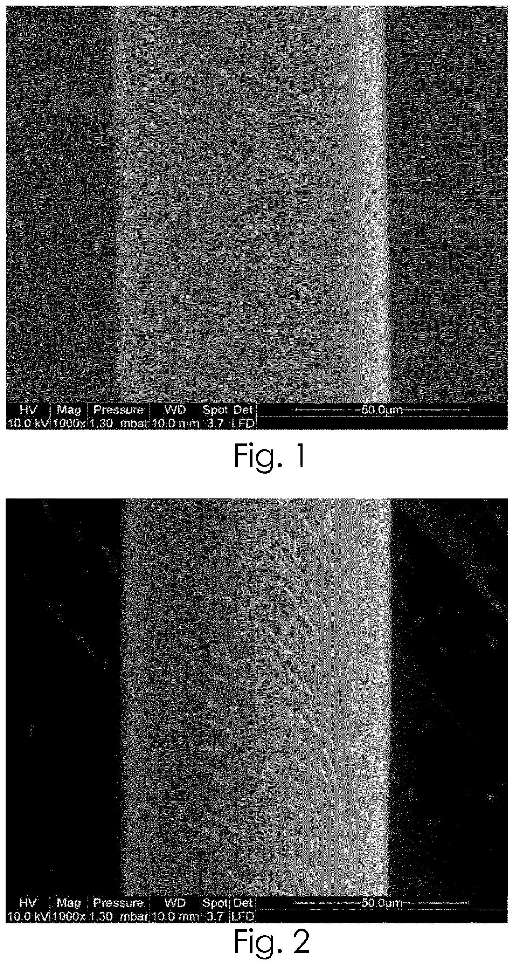

Tel qu'illustré par la Figure 1 (Essai 2357A.21), les écailles sont régulières, elles sont parallèles et ne sont pas décollées, et le cheveu est bien gainé.

Tel qu'illustré par la Figure 2 (Essai 2357A.22), on constate des trous dans les écailles signifiant un arrachement de cuticule. Certains endroits lisses démontrent l'absence de cuticule. On peut donc conclure à un peignage compliqué ayant entrainé l'arrachement des cuticules. Ce peignage compliqué s'explique par l'absence de gomme guar quaternisée dans la composition testée.

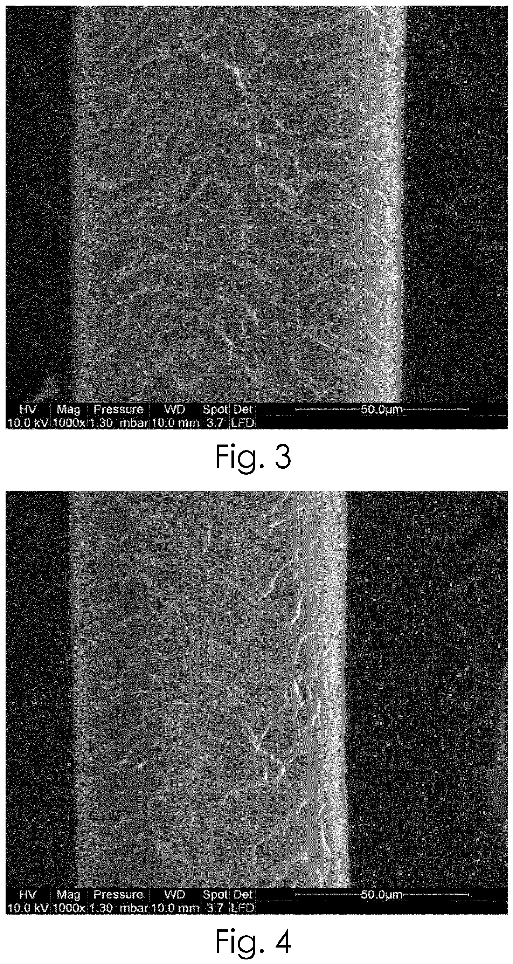

Tel qu'illustré par la Figure 3 (Essai 2357A.23), les écailles sont totalement décollées et désordonnées ; les bords blancs indiquent que les bords des cuticules sont plus proches du détecteur signifiant un décollement. Le cheveu n'est pas gainé.

Tel qu'illustré par la Figure 4 (Essai 2357A.24), les fibres ne sont pas belles, les écailles sont décollées et arrachées. Il reste beaucoup de débris d'écailles arrachées.

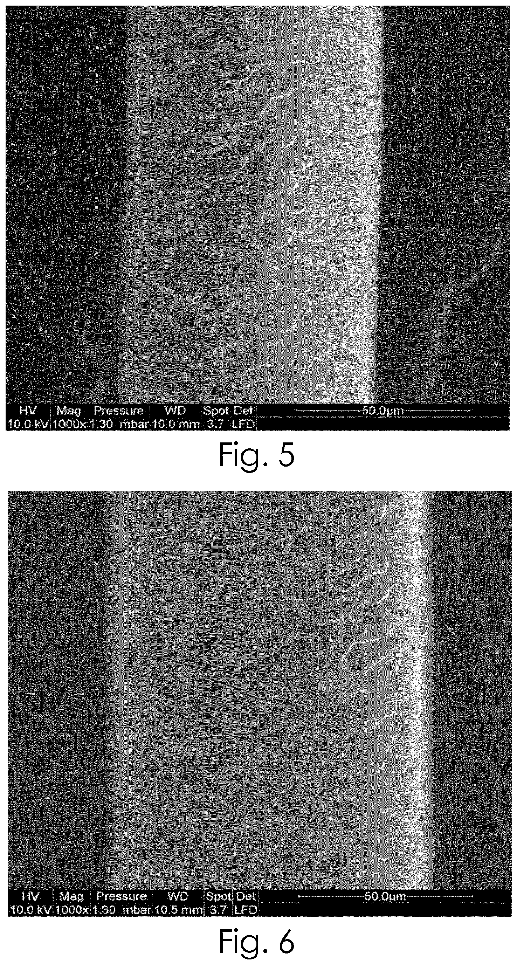

Tel qu'illustré par la Figure 5 (Essai 2357A.25), les écailles sont assez parallèles mais elles sont décollées, la fibre est moins gainée.

Tel qu'illustré par la Figure 6 (Essai 2357A.26), le cheveu est gainé, les écailles sont peu décollées et on peut même observer quelques dépôts d'huile de coco.

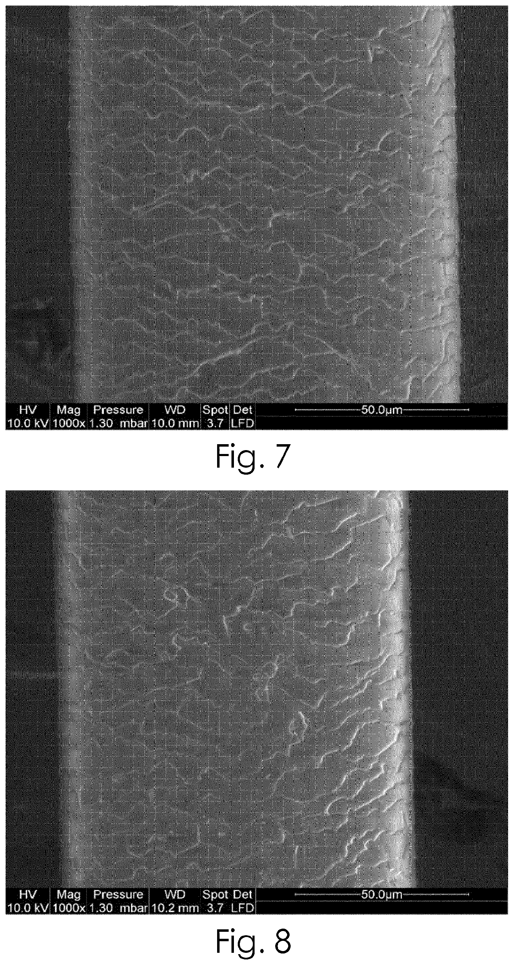

Tel qu'illustré par la Figure 7 (Essai 2357A.27), les écailles sont très soulevées, elles sont en train de se décoller par endroit, certaines sont dentelées, traduisant un manque d'hydratation.

Tel qu'illustré par la Figure 8 (Essai 2357A.28), les écailles sont décollées, il y a des débris d'écailles signifiant que des écailles ont été arrachées. Certaines écailles sont effacées, traduisant un nettoyage trop détergent.

Au global, sur la base des résultats obtenus dans le cadre de ce test, on peut conclure que :
l'aloé vera hydrate et protège les cuticules ;
l'huile de coco va gainer et protéger les cuticules des agressions extérieures quand son pourcentage est supérieur à 0,5% (p/p), par exemple de 1% ou 1,5% ;
les tensioactifs soufrés apparaissent être trop détergents, entraînant le détachement des cuticules ; et
la gomme guar quaternisée est nécessaire au bon démêlage des cheveux, protégeant ainsi les cuticules des agressions mécaniques.

### Exemple 3 : Test de mousse

Le pouvoir moussant d'une composition selon l'invention a été comparé à celle de produits contenant des tensioactifs soufrés et éthoxylés.

En effet, comme indiqué précédemment, une des contraintes techniques de cette invention a été de remplacer les tensioactifs soufrés et éthoxylés par des tensioactifs plus respectueux de la fibre capillaire tout en gardant une quantité et qualité de mousse acceptables dans le produit final.

Pour réaliser ces tests, la méthode de Ross-Miles a été utilisée. Le test de Ross-Miles est une expérience dont les conditions sont fixées par la norme ASTM D1173.

Il consiste à verser 200 mL d'une solution, concentrée à 1% en produit à tester, dans une ampoule à décanter.

Cette solution est ensuite vidée depuis une hauteur fixe (90 cm) dans une éprouvette graduée contenant déjà 50 mL de cette même solution.

Des mesures de hauteur de la colonne de mousse formée sont ensuite réalisées pour des temps de 30 secondes, 3 minutes et 5 minutes.

Ces solutions sont préparées avec de l'eau osmosée à pH=6,25.

Pour réaliser ces tests, la composition selon l'invention « témoin » (Essai 2357A.21, Tableau 2) a été utilisée et comparée avec une composition (Essai 2357A.29, Tableau 2) dans laquelle le mélange de tensioactifs non soufrés et non éthoxylés est remplacé par un tensioactif soufré (5,285% de sodium lauryl sulfate fourni par la société IFRACHIMIE S.A.S) pris en mélange avec de la cocamidopropyl betaine (4,736%).

Le pouvoir moussant de telles compositions a également été comparé avec des shampooings concurrents disponibles sur le marché contenant des tensioactifs soufrés et éthoxylés, à savoir :
Produit concurrent 1 :
   OGX - Nourishing + coconut milk shampoo^{®} contenant du sodium C14-16 olefin sulfonate (1-30%) ;
Produit concurrent 2 :
   GARNIER ULTRA-DOUX - Shampooing nourrissant sans silicone, lait de coco et macadamia^{®} contenant du sodium laureth sulfate (1-30%) ; et
Produit concurrent 3 :
   BOTANICALS Fresh Care - Shampooing Remède éclat Géranium^{®} contenant du sodium laureth sulfate (1-30%).

Les mesures ont été répétées 2 fois pour chaque produit. Les résultats obtenus sont détaillés ci-après :
Composition selon l'invention « Témoin » (Essai 2357A.21) :

**Tableau 3**

| Temps (s) | Hauteur de mousse (mL) |
|---|---|
| 30 | 90 |
| 180 (3 min) | 90 |
| 300 (5 min) | 90 |
| Hauteur moyenne (mL) | 90 |

**Tableau 4**

| Temps (s) | Hauteur de mousse (mL) |
|---|---|
| 30 | 85 |
| 180 (3 min) | 85 |
| 300 (5 min) | 85 |
| Hauteur moyenne (mL) | 85 |

Composition « Témoin » avec sulfate + bétaïne (Essai 2357A.29) :

**Tableau 5**

| Temps (s) | Hauteur de mousse (mL) |
|---|---|
| 30 | 100 |
| 180 (3 min) | 95 |
| 300 (5 min) | 95 |
| Hauteur moyenne (mL) | 97 |

**Tableau 6**

| Temps (s) | Hauteur de mousse (mL) |
|---|---|
| 30 | 90 |
| 180 (3 min) | 80 |
| 300 (5 min) | 80 |
| Hauteur moyenne (mL) | 83 |

### Produit concurrent 1 :

**Tableau 7**

| t (s) | Hauteur de mousse (mL) |
|---|---|
| 30 | 95 |
| 180 (3 min) | 90 |
| 300 (5 min) | 85 |
| Hauteur moyenne (mL) | 90 |

**Tableau 8**

| t (s) | Hauteur de mousse (mL) |
|---|---|
| 30 | 100 |
| 180 (3 min) | 100 |
| 300 (5 min) | 100 |
| Hauteur moyenne (mL) | 100 |

### Produit concurrent 2 :

**Tableau 9**

| t (s) | Hauteur de mousse (mL) |
|---|---|
| 30 | 70 |
| 180 (3 min) | 70 |
| 300 (5 min) | 70 |
| Hauteur moyenne (mL) | 70 |

**Tableau 10**

| t (s) | Hauteur de mousse (mL) |
|---|---|
| 30 | 80 |
| 180 (3 min) | 80 |
| 300 (5 min) | 75 |
| Hauteur moyenne (mL) | 78 |

### Produit concurrent 3 :

**Tableau 11**

| t(s) | Hauteur de mousse (mL) |
|---|---|
| 30 | 80 |
| 180 (3 min) | 70 |
| 300 (5 min) | 60 |
| Hauteur moyenne (mL) | 70 |

**Tableau 12**

| t (s) | Hauteur de mousse (mL) |
|---|---|
| 30 | 90 |
| 180 (3 min) | 90 |
| 300 (5 min) | 90 |
| Hauteur moyenne (mL) | 90 |

Les résultats ainsi obtenus mettent en évidence que le pouvoir moussant d'une composition selon l'invention comprenant des tensioactifs non soufrés ni éthoxylés se rapproche significativement des produits contenant des tensioactifs soufrés et éthoxylés.

Nous pouvons aussi constater que la mousse des produits comprenant des tensioactifs soufrés apparait être moins stable dans le temps que celle de la composition selon l'invention.

### Exemple 4 : Test coiffeur

Des tests ont également été réalisés avec un coiffeur professionnel indépendant dans les locaux du laboratoire Isatis situé à La Rochelle.

Le recrutement des panélistes sur lesquels les différents produits ont été testés s'est fait par le biais du laboratoire d'analyse sensorielle LEA SENSO^{®}. Les critères de recrutement étaient d'avoir des femmes, ayant les cheveux mi-longs afin d'avoir assez de matière pour évaluer les différents critères.

Les différents produits ont été codés par des codes à 3 chiffres, la composition selon l'invention «témoin » (Tableau 2), testée 7 fois, présentait un code différent pour chaque test afin que le coiffeur ne puisse pas l'identifier. L'ordre de présentation des produits était aléatoire c'est-à-dire que la composition «témoin» pouvait être aussi bien du côté gauche que du côté droit.

Pour réaliser ces tests, des applications par demi-tête ont été réalisées afin de pouvoir comparer les essais sur le même type de cheveu et sur des cheveux ayant exactement les mêmes conditions. Les quantités de produits appliquées ont été pesées afin d'appliquer les mêmes quantités des deux côtés.

Pour ce faire, le coiffeur a séparé les cheveux de la personne en deux par une raie au milieu.

Le coiffeur, a ensuite noté différents critères avec des notes allant de 0 à 10.

Les différents critères évalués ainsi que les bornes de notations sont les suivants :
- Volume de Mousse (0=très faible ; 10=très important) ;
- Qualité de Mousse (0=cassante, crissante ; 10=stable, douce, crémeuse) ;
- Rinçage (0=très difficile ; 10=très facile) ;
- Démêlage (0= très difficile ; 10=très facile) ;
- Brillance après séchage (0=ternes ; 10= très brillants) ;
- Volume après séchage (0=très plats ; 10=volume important) ;
- Douceur après séchage (0=très rêches ; 10=très doux) ;
- Souplesse après séchage (0=rigides ; 10=souples) ;
- Légèreté après séchage (0=lourds ; 10=légers).

Les effets de la composition selon l'invention « témoin » ont été respectivement comparés avec ceux des autres compositions mentionnées dans le Tableau 2.

### Exemple 4.A :

La composition selon l'invention «témoin» (Essai 2357A.21 = Référence 121) a été testée par rapport à la composition sans gomme guar quaternisée (Essai 2357A.22 = Référence 498), sur un type de cheveux fins, souples, colorés chimiquement.

Les résultats obtenus sont les suivants :

**Tableau 13**

| Référence | 121 (« Témoin ») | 498 |
|---|---|---|
| | Notes (/10) | |
| Volume de Mousse | 7 | 7 |
| Qualité de Mousse | 7 | 8 |
| Rinçage | 9 | 9 |
| Démêlage | 7 | 8 |
| Brillance après séchage | 10 | 7 |
| Volume après séchage | 7 | 9 |
| Douceur après séchage | 9 | 7 |
| Souplesse après séchage | 9 | 6 |
| Légèreté après séchage | 7 | 8 |

Le coiffeur n'a pas observé de grande différence en termes de mousse, de rinçage ou de démêlage lors de ce test.

En revanche, après séchage, le côté où la composition selon l'invention « témoin » a été appliquée est beaucoup plus brillant, les cheveux sont beaucoup plus doux, plus souples et plus gainés. Le fait qu'ils soient plus gainés entrainent un volume et une légèreté plus faible car le côté avec la composition sans gomme guar quaternisée (Référence 498) était très mousseux, donc plus volumineux et léger mais beaucoup moins beau.

On peut donc constater que la gomme guar quaternisée va apporter notamment de la brillance, douceur, gainage et souplesse aux cheveux, d'où son intérêt dans la composition selon l'invention.

### Exemple 4.B :

La composition selon l'invention «témoin» (Essai 2357A.21 = Référence 834) a été testée par rapport à la composition sans gomme guar quaternisée, remplacée par un ammonium quaternaire (Essai 2357A.23 = Référence 683), sur un type de cheveux naturels (sans coloration), frisés, secs, fins.

Les résultats obtenus sont les suivants :

**Tableau 14**

| Référence | 683 | 834 (« Témoin ») |
|---|---|---|
| | Notes (/10) | |
| Volume de Mousse | 7 | 7 |
| Qualité de Mousse | 7 | 7 |
| Rinçage | 8 | 8 |
| Démêlage | 6,5 | 8 |
| Brillance après séchage | 6 | 7 |
| Volume après séchage | 7 | 6 |
| Douceur après séchage | 6,5 | 8 |
| Souplesse après séchage | 9 | 9 |
| Légèreté après séchage | 9 | 9 |

Le coiffeur n'a pas observé de grande différence en termes de mousse et de rinçage. Le démêlage est plus facile sur le côté où la composition selon l'invention « témoin » a été appliquée.

Après séchage, les cheveux sont également plus doux du côté de la composition selon l'invention « témoin », les boucles sont mieux dessinées et le cheveu est plus gainé (donc moins volumineux, plus discipliné).

La panéliste a par ailleurs indiqué avoir ressenti des démangeaisons et des tiraillements du côté où la Référence 683 a été appliquée. Nous pouvons supposer que ces démangeaisons sont causées par la présence du Cetrimonium Chloride, qui est une matière suspectée irritante.

La gomme guar quaternisée comprise dans la composition selon l'invention a un rôle essentiel et ne peut être avantageusement remplacée par un autre ammonium quaternaire.

### Exemple 4.C :

La composition selon l'invention «témoin» (Essai 2357A.21 = Référence 148) a été testée par rapport à la composition sans huile de noix de coco (Essai 2357A.24 = Référence 946), sur un type de cheveux naturels avec quelques mèches.

Les résultats obtenus sont les suivants :

**Tableau 15**

| Référence | 148 (« Témoin ») | 946 |
|---|---|---|
| | Notes (/10) | |
| Volume de Mousse | 7 | 10 |
| Qualité de Mousse | 7 | 9 |
| Rinçage | 9 | 9 |
| Démêlage | 8 | 6 |
| Brillance après séchage | 9 | 9 |
| Volume après séchage | 7 | 9 |
| Douceur après séchage | 9 | 7 |
| Souplesse après séchage | 9 | 8 |
| Légèreté après séchage | 8 | 9 |

Dans cet exemple, nous avons observé une grande différence de mousse, le shampooing sans huile de noix de coco moussant bien mieux. Les huiles végétales ont, en effet, tendance à casser la mousse.

En revanche, concernant le démêlage, nous pouvons constater que l'huile de noix de coco va aider notablement à faciliter le démêlage.

Après séchage, les cheveux sont également plus doux et plus gainés (moins volumineux donc) du côté où la composition selon l'invention « témoin » contenant de l'huile de noix de coco a été appliquée.

Il a par ailleurs été observé beaucoup de frisottis du côté où la Référence 946 a été appliquée.

### Exemple 4.D :

La composition selon l'invention «témoin» (Essai 2357A.21 = Référence 117) a été testée par rapport à la composition avec 0,5% d'huile de noix de coco (Essai 2357A.25 = Référence 432), sur un type de cheveux naturels, méchés, gras.

Les résultats obtenus sont les suivants :

**Tableau 16**

| Référence | 117 (« Témoin ») | 432 |
|---|---|---|
| | Notes (/10) | |
| Volume de Mousse | 8 | 6 |
| Qualité de Mousse | 9 | 5 |
| Rinçage | 8 | 8 |
| Démêlage | 8 | 7 |
| Brillance après séchage | 6 | 6 |
| Volume après séchage | 9 | 6 |
| Douceur après séchage | 8 | 8 |
| Souplesse après séchage | 9 | 7 |
| Légèreté après séchage | 9 | 7 |

On peut observer une grande différence de mousse, qu'il est préférable de ne pas exploiter, la panéliste ayant un cuir chevelu très gras ce qui a eu pour effet de casser la mousse durant les shampooings.

Concernant le démêlage, le coiffeur a néanmoins constaté une amélioration du démêlage lorsque la composition selon l'invention « témoin » contenant 1,5% d'huile de noix de coco a été appliquée sur le témoin.

Après séchage, les cheveux sont équivalents en termes de brillance et de douceur. Les cheveux traités avec la composition « témoin » sont cependant plus légers, plus souples et plus volumineux.

Ce test démontre la nécessité d'ajouter l'huile de noix de coco à un pourcentage significatif de plus de 0,5% dans la composition selon l'invention.

### Exemple 4.E :

La composition selon l'invention «témoin» (Essai 2357A.21 = Référence 457) a été testée par rapport à la composition avec 1% d'huile de noix de coco (Essai 2357A.26 = Référence 591), sur un type de cheveux colorés chimiquement, épais.

Les résultats obtenus sont les suivants :

**Tableau 17**

| Référence | 591 | 457 (« Témoin ») |
|---|---|---|
| | Notes (/10) | |
| Volume de Mousse | 7 | 7 |
| Qualité de Mousse | 7 | 8 |
| Rinçage | 8 | 8 |
| Démêlage | 6,5 | 8 |
| Brillance après séchage | 6 | 6 |
| Volume après séchage | 6 | 6 |
| Douceur après séchage | 8 | 8 |
| Souplesse après séchage | 3 | 3 |
| Légèreté après séchage | 3 | 3 |

Peu de différences ont été observées au global entre ces deux compositions.

On peut noter une petite différence au niveau de la qualité de mousse, la mousse étant plus crémeuse et plus douce dans la composition selon l'invention « témoin » contenant plus d'huile de noix de coco (1,5%).

Le démêlage est également facilité plus la quantité d'huile de noix de coco augmente.

Les résultats de souplesse et légèreté obtenus s'expliquent par le type de cheveux de la panéliste. En effet, cette dernière avait des cheveux très épais, ne pouvant donc pas être souples ni légers.

Ce test a permis de montrer qu'il est avantageux d'augmenter la concentration en huile de noix de coco : plus on augmente la concentration en huile de noix de coco (*i*.e. 1,5%), plus la mousse est douce et crémeuse et plus le démêlage est facilité.

### Exemple 4.F :

La composition selon l'invention «témoin» (Essai 2357A.21 = Référence 701) a été testée par rapport à la composition sans aloé vera (Essai 2357A.27 = Référence 252), sur un type de cheveux colorés chimiquement, mousseux.

Les résultats obtenus sont les suivants :

**Tableau 18**

| Référence | 701 (« Témoin ») | 252 |
|---|---|---|
| | Notes (/10) | |
| Volume de Mousse | 7 | 6 |
| Qualité de Mousse | 8 | 7 |
| Rinçage | 8 | 8 |
| Démêlage | 6 | 8 |
| Brillance après séchage | 9 | 9 |
| Volume après séchage | 8 | 9 |
| Douceur après séchage | 9 | 7 |
| Souplesse après séchage | 9 | 8 |
| Légèreté après séchage | 7 | 9 |

L'ajout d'aloé vera semble aider à stabiliser la mousse. Il n'a pas d'impact sur le rinçage et semble cependant légèrement compliquer le démêlage.

Après séchage, les cheveux sont plus doux, plus souples et plus gainés (donc moins volumineux et moins légers) du côté où la composition selon l'invention « témoin » contenant de l'aloé vera a été appliquée.

Ce test permet de valider les effets de l'aloé vera sur cheveux secs, de confirmer son apport de douceur et de souplesse et mettre en évidence ses propriétés avantageuses dans la composition selon l'invention.

### Exemple 4.G :

La composition selon l'invention «témoin» (Essai 2357A.21 = Référence 384) a été testée par rapport à la composition avec uniquement un tensioactif soufré (Essai 2357A.29 = Référence 163), sur un type de cheveux naturels, secs.

Les résultats obtenus sont les suivants :

**Tableau 19**

| Référence | 163 | 384 (« Témoin ») |
|---|---|---|
| | Notes (/10) | |
| Volume de Mousse | 10 | 8 |
| Qualité de Mousse | 9 | 8 |
| Rinçage | 9 | 9 |
| Démêlage | 10 | 8 |
| Brillance après séchage | 7 | 9 |
| Volume après séchage | 9 | 7 |
| Douceur après séchage | 7 | 9 |
| Souplesse après séchage | 7 | 9 |
| Légèreté après séchage | 8 | 7 |

Comme attendu, les tensioactifs soufrés ont apporté bien plus de mousse que les tensioactifs non soufrés et non éthoxylés utilisés dans la composition selon l'invention « témoin ». Il semblerait qu'ils aient également aidé au démêlage.

Toutefois, après séchage, les cheveux traités par la composition selon l'invention « témoin » sont bien plus brillants, plus doux, plus souples et plus gainés (donc moins de volume et de légèreté).

De plus, le côté traité avec la composition comprenant le tensioactif soufré (sulfate) était rempli de frisottis et les cheveux n'étaient pas du tout gainés et paraissaient secs visuellement.

Les tensioactifs soufrés tels que les sulfates apportent donc plus de mousse à la composition que dans la composition selon l'invention. Néanmoins, le résultat sur cheveux secs est flagrant, visible directement et bien moins satisfaisant. Les tensioactifs soufrés vont en effet apporter un côté rêche et terne aux cheveux.

En conclusion, ces tests ont permis de confirmer que :
l'aloé vera apporte de la douceur, souplesse et gainage aux cheveux ;
l'huile de coco aide au démêlage. Elle apporte également de la douceur et gainage aux cheveux quand elle est utilisée à un pourcentage significatif (supérieur à 0,5% (p/p), par exemple d'au moins 1%) ;
les tensioactifs soufrés, très bons moussants, peut-être même trop détergents, vont ternir et apporter un côté rêche aux cheveux. Ils sont de manière inattendue avantageusement remplacés par des tensioactifs non soufrés et non éthoxylés qui permettent en outre d'obtenir un pouvoir moussant et une stabilité satisfaisants ; et
la gomme guar quaternisée apporte de la brillance, douceur, gainage et souplesse aux cheveux. Elle n'apparait en outre pouvoir être avantageusement remplacée par un autre ammonium quaternaire.

## Revendications

1. Composition cosmétique destinée à être appliquée sur les cheveux et le cuir chevelu comprenant des extraits d'aloé vera (*Aloe barbadensis*) et de fruit de cocotier (*Cocos nucifera*), **caractérisée en ce qu'**elle se présente sous forme d'une émulsion moussante comprenant, dans un milieu cosmétiquement acceptable :
- un extrait d'aloé vera choisi parmi une eau, un jus et/ou de la poudre d'aloé vera ;
- entre 1% et 20% en poids du poids total de la composition (p/p) d'une huile de noix de coco ;
- au moins un tensioactif non soufré et non éthoxylé et ne comprend pas de tensioactif soufré et/ou éthoxylé ; et
- au moins un agent conditionneur cationique issu de gomme guar (*Cyamopsis tetragonoloba*).

2. Composition selon la revendication 1, **caractérisée en ce qu'**elle comprend de la poudre de feuilles d'aloé vera à une concentration comprise entre 0,01% et 2% en poids du poids total de la composition (p/p) et/ou du jus de feuilles d'aloé vera à une concentration comprise entre 0,5% et 15% en poids du poids total de la composition (p/p).

3. Composition selon l'une des revendications précédentes, **caractérisée en ce que** le tensioactif non soufré et non éthoxylé est choisi parmi la cocamidopropyl betaine, le decyl glucoside, le sodium cocoyl glutamate, le lauryl glucoside, le coco glucoside, le sodium lauroyl glutamate, le sodium olivoyl glutamate, et les tensioactifs dérivés de glucamide tels que le cocoyl méthyle glucamide ou le sunfloweroyl méthyle glucamide, pris seul ou en mélange, préférentiellement un mélange de cocamidopropyl betaine, decyl glucoside et sodium cocoyl glutamate.

4. Composition selon la revendication 3, **caractérisée en ce qu'**elle comprend respectivement, en combinaison :
entre 0,5 et 20% (p/p) de cocamidopropyl betaine,
entre 0,5% et 20% (p/p) de decyl glucoside, et
entre 0,25% et 10% (p/p) de sodium cocoyl glutamate.

5. Composition selon l'une des revendications précédentes, **caractérisée en ce que** l'agent conditionneur cationique issu de gomme guar est un dérivé de gomme guar quaternisée, préférentiellement un chlorure de guar hydroxypropyltriméthylammonium.

6. Composition selon l'une des revendications précédentes, **caractérisée en ce qu'**elle comprend l'agent conditionneur cationique issu de gomme guar à une concentration comprise entre 0,05% et 1% en poids du poids total de la composition (p/p).

7. Composition selon l'une des revendications précédentes, **caractérisée en ce qu'**elle comprend en outre au moins un gélifiant anionique choisi parmi la gomme xanthane, la gomme caroube, la gomme gellane, la cellulose, la pectine, la bentonite, les carraghénanes, préférentiellement la gomme xanthane.

8. Composition selon la revendication 7, **caractérisée en ce qu'**elle comprend l'agent gélifiant anionique à une concentration comprise entre 0,1% et 2% en poids du poids total de la composition (p/p).

9. Composition selon l'une des revendications précédentes, **caractérisée en ce qu'**elle comprend en outre un agent hydratant ou apaisant choisi parmi l'acide hyaluronique, le sodium PCA, la glycérine et autres dérivés du glycérol ou un polysaccharide tel qu'un polysaccharide riche en galactomannanes, par exemple la gomme tara (*caesalpina spinosa*).

10. Composition selon l'une des revendications précédentes, **caractérisée en ce qu'**elle comprend en outre une autre huile végétale choisie parmi l'huile de ricin, argan, jojoba, amande douce, karité, avocat, carotte, noyaux d'abricot, germe de blé, nigelle, moutarde, bourrache, noisette, macadamia, abyssinie, sésame, onagre, figue de barbarie, pépins de raisin, carthame, cameline, karanja, camélia, chia, lin, mangue, baobab, brocolis, andiroba, babassu, dattier du désert, monoï, moringa, prise seule ou en mélange.

11. Composition selon l'une des revendications précédentes, **caractérisée en ce qu'**elle comprend en outre un autre agent conditionneur capillaire choisi parmi l'hydroxypropyltrimonium honey, le brassicamodipropyl dimethylamine, le brassicyl isoleucinate esylate et le brassicyl valinate esylate, pris seul ou en mélange.

12. Procédé de préparation d'une composition selon l'une des revendications 1 à 11, **caractérisé en ce qu'**il comprend les étapes suivantes selon lesquelles :
a) on mélange le ou les extraits d'aloé vera choisi(s) parmi une eau, un jus et/ou de la poudre d'aloé vera dans de l'eau,
b) on disperse au moins un agent conditionneur cationique issu de gomme guar dans le mélange obtenu en a),
c) après neutralisation, on laisse un gel aqueux se former par agitation supérieure à 1000 tours/minute sous émulseur,
d) éventuellement, on disperse un gélifiant anionique par agitation supérieure à 1000 tours/minute sous émulseur et chauffage à une température supérieure à 50°C,
e) entre 1% et 20% d'huile de noix de coco en poids du poids total de la composition (p/p) est chauffé à une température supérieure à 50°C puis est ajouté au gel aqueux obtenu en c) ou d) sous émulseur, et
f) un tensioactif non soufré et non éthoxylé est ajouté à une température inférieure à 30°C sous agitation inférieure à 1000 tours/minutes pour obtenir une émulsion moussante.

13. Utilisation cosmétique d'une composition selon l'une des revendications 1 à 11 pour le lavage ou le soin des cheveux.

14. Procédé de traitement cosmétique des cheveux, **caractérisé en ce qu'**il consiste à appliquer sur les cheveux une quantité efficace d'une composition selon l'une des revendications 1 à 11 puis à effectuer éventuellement un rinçage à l'eau.

## Patentansprüche

1. Kosmetische Zusammensetzung, die auf das Haar und die Kopfhaut aufgetragen werden soll und Extrakte aus Aloe Vera (*Aloe borbodensis*) und Kokosnussfrüchten (*Cocos nucifero*) umfasst, **dadurch gekennzeichnet ist, dass** sie in Form einer schaumbildenden Emulsion vorliegt, in einem kosmetisch verträglichen Medium umfassend:
- einen Aloe-Vera-Extrakt, ausgewählt aus Wasser, einem Saft und / oder Aloe-Vera-Pulver;
- Kokosnussöl, in einer Konzentration zwischen 1 Gew.-% und 20 Gew.-% des Gesamtgewichts der Zusammensetzung (Prozentpunkt);
- mindestens ein nicht schwefelhaltiges und nicht ethoxyliertes Tensid und kein schwefelhaltiges und / oder ethoxyliertes Tensid enthält; und
- mindestens ein kationisches Konditionierungsmittel aus Guarkernmehl (*Cyomopsis tetrogonolobo*).

2. Zusammensetzung nach Anspruch 1, **dadurch gekennzeichnet, dass** sie Pulver aus Aloe-Vera-Blättern in einer Konzentration zwischen 0,01 Gew.-% bis 2 Gew.-% des Gesamtgewichts der Zusammensetzung (w/w) und / oder Saft aus Aloe-Vera-Blättern in einer Konzentration von zwischen 0,5 und 15 Gew.-% des Gesamtgewichts der Zusammensetzung (w/w) umfasst.

3. Zusammensetzung nach einem der vorangehenden Ansprüche, **dadurch gekennzeichnet, dass** das schwefelfreie und nicht ethoxylierte Tensid aus Cocamidopropylbetain, Decylglucosid, Natriumcocoylglutamat, Laurylglucosid, Kokosglucosid, Natriumlauroylglutamat, Natriumolivoylglutamat, und die Tenside, die von Glucamid abgeleitet sind, wie Cocoylmethylglucamid oder Sonnenblumenoylmethylglucamid, allein oder als Mischung, vorzugsweise eine Mischung aus Cocamidopropylbetain, Decylglucosid und Natriumcocoylglutamat ausgewählt werden.

4. Zusammensetzung nach Anspruch 3, **dadurch gekennzeichnet, dass** sie jeweils in Kombination umfasst:
zwischen 0,5 % und 20 % (w/w) Cocamidopropylbetain,
zwischen 0,5 % und 20 % (w/w) Decylglucosid, und
zwischen 0,25 % und 10 % (w/w) Natriumcocoylglutamat.

5. Zusammensetzung nach einem der vorhergehenden Ansprüche, **dadurch gekennzeichnet, dass** das von Guargummi abgeleitete kationische Konditionierungsmittel ein Derivat von quaternisiertem Guargummi ist, vorzugsweise einem Hydroxypropyltrimethylammoniumguarchlorid.

6. Zusammensetzung nach einem der vorhergehenden Ansprüche, **dadurch gekennzeichnet, dass** sie das aus Guargummi erhaltene kationische Konditionierungsmittel in einer Konzentration zwischen 0,05 Gew.-% und 1 Gew.-% des Gesamtgewichts der Zusammensetzung (w/w) umfasst.

7. Zusammensetzung nach einem der vorhergehenden Ansprüche, **dadurch gekennzeichnet, dass** Guargummi ist sie ferner mindestens ein anionisches Geliermittel umfasst, ausgewählt aus Xanthangummi, Johannisbrotkernmehl, Gellangummi, Cellulose, Pektin, Bentonit, Carrageenanen, vorzugsweise Xanthangummi.

8. Zusammensetzung nach Anspruch 7, **dadurch gekennzeichnet, dass** sie das anionische Geliermittel in einer Konzentration zwischen 0,1 Gew.-% und 2 Gew.-% des Gesamtgewichts der Zusammensetzung (w/w).

9. Zusammensetzung nach einem der vorhergehenden Ansprüche, **dadurch gekennzeichnet, dass** sie ferner ein Feuchtigkeits- oder Beruhigungsmittel umfasst, das aus Hyaluronsäure, Natrium-PCA, Glycerin und anderen Glycerinderivaten oder einem Polysaccharid wie einem an Galactomannanen reichen Polysaccharid, beispielsweise Taragummi (*caesalpina spinosa*) ausgewählt ist.

10. Zusammensetzung nach einem der vorhergehenden Ansprüche, **dadurch gekennzeichnet, dass** sie ferner ein weiteres Pflanzenöl enthält, ausgewählt aus Ölen wie Rizinusöl, Argon, Jojoba, Süßmandel, Shea, Avocado, Karotte, Aprikosenkern, Weizenkeimen, Nigella, Senf, Borretsch, Haselnuss, Macadamia, Abessinien, Sesam, Nachtkerze, Kaktusfeigen, Traubenkern, Saflor, Camelina, Karanja, Kamelien, Chia, Flachs, Mango, Baobab, Brokkoli, Andiroba, Babassu, Wüstendatteln, Monoi, Moringa, allein oder als Mischung genommen.

11. Zusammensetzung nach einem der vorhergehenden Ansprüche, **dadurch gekennzeichnet, dass** sie ferner ein weiteres Haarkonditionierungsmittel umfasst, ausgewählt aus Hydroxypropyltrimoniumhonig, Brassicamodipropyl-Dimethylamin, Brassicyl-Isoleucinat-Esylat und Brassicyl-Valinat-Esylat, allein oder als Mischung genommen.

12. Verfahren zur Herstellung einer Zusammensetzung nach einem der Ansprüche 1 bis 11, **dadurch gekennzeichnet, dass** es die folgenden Schritte umfasst, wobei
a) die Aloe Vera-Extrakt/e, ausgewählt aus einem Wasser, einem Saft und/oder Pulver von Aloe Vera in Wasser gemischt werden,
b) mindestens ein aus Guargummi gewonnenes kationisches Konditionierungsmittel in der in a) erhaltenen Mischung dispergiert ist,
c) nach der Neutralisation durch Rühren bei mehr als 1000 U/min unter einem Emulgator ein wässriges Gel zur Entstehung gebracht wird,
d) gegebenenfalls ein anionisches Geliermittel durch Rühren bei mehr als 1000 U/min bei Anwesenheit eines Emulgators und durch Erwärmen auf eine Temperatur von mehr als 50°C dispergiert wird,
e) zwischen 1 Gew.-% und 20 Gew.-% Kokosnussöl, bezogen auf das Gesamtgewicht der Zusammensetzung (w/w), auf eine Temperatur über 50°C erhitzt und dann in Anwesenheit eines Emulgators zu dem in c) oder d) erhaltenen wässrigen Gel gegeben wird, und
f) ein nicht-schwefelhaltiges und nicht-ethoxyliertes Tensid bei einer Temperatur unter 30°C unter Rühren bei weniger als 1000 U/min zugegeben wird, um eine schäumende Emulsion zu erhalten.

13. Kosmetische Verwendung einer Zusammensetzung nach einem der Ansprüche 1 bis 11 zum Waschen oder Pflegen der Haare.

14. Verfahren zur kosmetischen Behandlung der Haare, **dadurch gekennzeichnet, dass** es darin besteht, auf die Haare eine wirksame Menge einer Zusammensetzung nach einem der Ansprüche 1 bis 11 aufzutragen und dann gegebenenfalls mit Wasser zu spülen.

## Claims

1. A cosmetic composition for application to hair and scalp comprising aloe vera (*Aloe barbadensis*) and coconut fruit (*Cocos nucifera*) extracts, **characterised in that** it is in the form of a foaming emulsion comprising, in a cosmetically acceptable medium:
- an aloe vera extract chosen from water, juice and/or powder of aloe vera;
- between 1% and 20% by weight of the total weight of the composition (w/w) of coconut oil;
- at least one non-sulfurised and non-ethoxylated surfactant and does not comprise a sulfurised and/or ethoxylated surfactant; and
- at least one cationic conditioning agent derived from guar gum (*Cyamopsis tetragonoloba*).

2. The composition according to claim 1, **characterised in that** it comprises aloe vera leaf powder at a concentration of between 0.01% and 2% by weight of the total weight of the composition (w/w) and/or the aloe vera leaf juice at a concentration of between 0.5% and 15% by weight of the total weight of the composition (w/w).

3. The composition according to any one of the preceding claims, **characterised in that** the non-sulfurised and non-ethoxylated surfactant is chosen from cocamidopropyl betaine, decyl glucoside, sodium cocoyl glutamate, lauryl glucoside, coco glucoside, sodium lauroyl glutamate, sodium olivoyl glutamate, and the surfactants derived from glucamide such as cocoyl methyl glucamide or sunfloweroyl methyl glucamide, taken alone or as a mixture, preferably a mixture of cocamidopropyl betaine, decyl glucoside and sodium cocoyl glutamate.

4. The composition according to claim 3, **characterised in that** it comprises, in combination:
between 0.5 and 20% (w/w) of cocamidopropyl betaine,
between 0.5% and 20% (w/w) of decyl glucoside, and
between 0.25% and 10% (w/w) of sodium cocoyl glutamate.

5. The composition according to any one of the preceding claims, **characterised in that** the cationic conditioning agent derived from guar gum is a quaternised guar gum derivative, preferably hydroxypropyltrimethylammonium guar chloride.

6. The composition according to any one of the preceding claims, **characterised in that** it comprises the cationic conditioning agent derived from guar gum at a concentration of between 0.05% and 1% by weight of the total weight of the composition (w/w).

7. The composition according to any one of the preceding claims, **characterised in that** it further comprises at least one anionic gelling agent chosen from xanthan gum, locust bean gum, gellan gum, cellulose, pectin, bentonite, carrageenans, preferably xanthan gum.

8. The composition according to claim 7, **characterised in that** it comprises the anionic gelling agent at a concentration of between 0.1% and 2% by weight of the total weight of the composition (w/w).

9. The composition according to any one of the preceding claims, **characterised in that** it further comprises a moisturising or soothing agent chosen from hyaluronic acid, sodium PCA, glycerin and other derivatives of glycerol or a polysaccharide such as a polysaccharide rich in galactomannans, for example tara gum (*Caesalpina spinosa*).

10. The composition according to any one of the preceding claims, **characterised in that** it further comprises another vegetable oil chosen from castor oil, argan, jojoba, sweet almond, shea, avocado, carrot, apricot kernel, wheatgerm, nigella, mustard, borage, hazelnut, macadamia, Abyssinian oil, sesame, evening primrose, prickly pear, grape seed, safflower, camelina, karanja, camellia, chia, flax, mango, baobab, broccoli, andiroba, babassu, desert date palm, monoï; moringa, taken alone or as a mixture.

11. The composition according to any one of the preceding claims, **characterised in that** it further comprises another hair conditioning agent chosen from hydroxypropyltrimonium honey, brassicamidopropyl dimethylamine, brassicyl isoleucinate esylate and brassicyl valinate esylate, taken alone or as a mixture.

12. A method for preparing a composition according to any one of claims 1 to 11, **characterised in that** it comprises the following steps, according to which:
a) the aloe vera extract(s) chosen from water, juice and/or powder of aloe vera is mixed in water,
b) at least one cationic conditioning agent derived from guar gum is dispersed in the mixture obtained in a),
c) after neutralisation, an aqueous gel is allowed to form by stirring above 1000 rpm under an emulsifier,
d) optionally, an anionic gelling agent is dispersed by stirring above 1000 rpm under an emulsifier and heated to a temperature above 50°C,
e) between 1% and 20% of coconut oil by weight of the total weight of the composition (w/w) is heated to a temperature above 50°C and then added to the aqueous gel obtained in c) or d) under an emulsifier, and
f) a non-sulfurised and non-ethoxylated surfactant is added at a temperature of less than 30°C with stirring of less than 1000 rpm to obtain a foaming emulsion.

13. Cosmetic use of a composition according to any one of claims 1 to 11 for washing or caring for hair.

14. A method for cosmetic treatment of hair, **characterised in that** it consists in applying to the hairan effective amount of a composition according to any one of claims 1 to 11 and then optionally rinsing with water.
